# EUROPÄISCHE PATENTSCHRIFT

(11) **EP 1 953 215 B1**
(45) Veröffentlichungstag und Bekanntmachung des Hinweises auf die Patenterteilung: **28.07.2010**
(21) Anmeldenummer: 08001264.4
(22) Anmeldetag: 24.01.2008
(51) Int. Cl.: C11D 3/20, C11D 3/22, C11D 3/43, C11D 10/04, C11D 17/00, A61L 9/05

(54) **Transparentes Toilettenreinigungs- und Beduftungsmittel**
Transparent toilet cleaning and deodorization composition
Composition transparente de nettoyage et de desodorisation pour les toilettes

(30) Priorität: 31.01.2007 DE 102007005617
(43) Veröffentlichungstag der Anmeldung: 06.08.2008
(73) Patentinhaber: Buck-Chemie GmbH, 71083 Herrenberg (DE)
(72) Erfinder: Leipold, Joachim, Dr., 72760 Reutlingen (DE); Jaeschke, Edgar, 70794 Filderstadt (DE); Fritz, Matthias, 72810 Gomaringen (DE)
(74) Vertreter: Mammel, Ulrike

(56) Entgegenhaltungen:
- EP-A- 1 219 701
- WO-A-01/11001
- WO-A-96/04360
- US-A1- 2001 044 394
- DATABASE WPI Week 198915 Derwent Publications Ltd., London, GB; AN 1989-112337 XP002479280 & JP 01 060696 A (NIPPON OILS & FATS CO LTD) 7. März 1989 (1989-03-07)

## Beschreibung

Die vorliegende Erfindung betrifft ein transparentes stückförmiges Toilettenreinigungs- und Beduftungsmittel.

Im Stand der Technik ist eine Vielzahl von Toilettenreinigungsmitteln bekannt, die neben Tensiden und Duftstoffen auch hohe Anteile an anorganischen Salzen umfassen. Diese Salze dienen als Füllstoffe und zur Steuerung der Auflösegeschwindigkeit, da sich die Mittel nach und nach auflösen sollen. Die bekannten Mittel sind undurchsichtig oder opak und werden üblicherweise mittels Extrusion hergestellt. Je nach Formulierung können sie sowohl im Spülkasten einer Toilette als auch als Rim-Block eingesetzt werden.

In der Anwendung als Rim-Block werden die Mittel in einem an dem Toilettenrand befestigbaren Behältnis aufbewahrt, in dem sie von Spülwasser überströmt werden und sich unter Freisetzung der Wirkstoffe nach und nach auflösen. In der Regel liegen die Spülzahlen der Rimblocks wenigstens bei 150 bis 200 Spülungen/Mittel.

Nachteilig ist bei den bekannten Toilettenreinigungsmitteln der hohe Gehalt an Salzen wie NaCl oder Na₂SO₄, die als Füllstoffe und zur Steuerung der Auflösegeschwindigkeit zugegeben werden. Auch wird den Mitteln Natriumcumolsulfonat, das als Lösungsvermittler (Hydrotropikum) dient, zugegeben, um den Toilettenstein während seiner Nutzungszeit (≥ 250 Spülungen) möglichst konstant zu verbrauchen.

Diese Stoffe, deren Anteil mehr als 50 Gew.% des Mittels ausmachen kann, führen zu einer unnötigen Belastung des Abwassers und Schädigung der Umwelt.

In den letzten Jahren hat sich zudem der Wunsch der Verbraucher nach optisch ansprechenden, dekorativen Toilettenreinigungsmitteln verstärkt, wobei der Trend zu transparenten gelförmigen Systemen geht. Solche transparente gelförmige Systeme, die beispielsweise in der DE 197 10 635 A1 beschrieben sind und u.a. Tenside, Gelbildner, Duftstoffe und Lösemittel umfassen, zeichnen sich zwar durch eine permanente und ausreichende Raumbeduftung und eine gute Schaumbildung aus, die erforderliche Reinigungsleistung kann mit diesen Mitteln infolge des hohen Lösemittelanteils jedoch nicht erzielt werden.

Um ein Toilettenreinigungsmittel bereit zu stellen, das sowohl eine gute Reinigungsleistung als auch eine ausreichende und permanente Raumbeduftung bewirkt, wurde in der EP 1 418 225 A1 ein zweiphasiges Mittel aus einem Reinigungsmittelformkörper mit Tensiden für die Reinigung und einer Gelphase mit Duftstoffen für die Beduftung vorgeschlagen.

Dieses Mittel zeigt zwar eine gute Reinigung und Beduftung, allerdings ist die Herstellung, die sowohl auf einer Extrusion als auch einer zusätzlichen Gelherstellung basiert, aufwändig. Weiterhin werden infolge des Salzgehaltes der Reinigungsmittelformkörperphase die Abwässer unnötig mit Salz belastet.

Aus der Patentanmeldung US 2001/0044394 A1 ist ein zweischichtiger dekorativer Toilettenreinigungsblock bekannt, der neben einer eingefärbten transparenten Seifenformulierung eine opake syndet-basierte Seifenzusammensetzung umfasst.

Die Seifenausgangsformulierung umfasst neben Tensiden und den Fettsäuren Stearin- und Myristinsäure, Glycerin, Natriumhydroxid sowie Propylenglykol, Wasser und Sorbitol, so dass der Zusatz von anorganischen Salzen bei dieser Formulierung vermieden wird. Die Seifenphase des zweischichtigen Toilettenblocks wirkt vor dem Hintergrund der opaken Syndet-Phase im Wesentlichen transparent, und man kann auch durch eine 0,63 cm dicke Schicht dieser Seifenphase noch eine 14 Punkt-Schrift lesen.

Die Transparenz einer jeden Schicht nimmt jedoch mit zunehmender Schichtdicke ab. In dickeren Schichten, die üblicherweise in Toilettenreinigungsmitteln vorliegen, erscheinen die aus der US 2001/0044394 A1 bekannten Seifenformulierungen jedoch nicht mehr transparent sondern eher milchig.

Aus der WO 96/04360 sind transparente Reinigungsmittelstücke bekannt, die jedoch zur Körperpflege dienen. Diese eigenen sich infolge der geringen Parfümierung nicht zur Beduftung des Toilettenbereichs. Der geringe Parfümanteil trägt jedoch zu einer höheren Härte und zu einer besseren Transparenz des Mittels bei. Die Körperpflegeseife weist zudem einen relativ hohen Anteil an Carbonsäuren auf, was für die Härte des Mittels wünschenswert ist, jedoch die unerwünschte Bildung von Kalkseifen mit sich bringt.

Weiterhin lehrt die WO 98/51773 eine transparente Seife, die 30 Gew.% Carbonsäuren, Sucrose, Sorbitol, Parfüm und Wasser umfasst. Nachteilig an dieser Seife ist, dass diese relativ weich ist, zudem erst 10 bis 12 Wochen "reifen" muss, um die gewünschte Transparenz zu erreichen und auch noch einen hohen Anteil an Kalkseife bildenden Carbonsäuren umfasst.

Transparente Seifenstücke sind weiterhin aus der EP-A-1219701 und der WO-A-0111001 bekannt. Als Transparenz gebende Zusätze für Seifen offenbart JP-A-1161099 (WPI) Sucrose, Glycerin, Sorbitol und Propylenglycol.

Die Aufgabe der vorliegenden Erfindung besteht darin, ein stückförmiges Toilettenreinigungs- und Beduftungsmittel anzugeben, das umweltfreundlich und einfach herstellbar ist und sich durch eine hohe Transparenz, hinreichende Beduftung und Härte auszeichnet und Spülzahlen von wenigstens 150 aufweist.

Diese Aufgabe wird durch ein Toilettenreinigungsmittel gelöst, das 10 bis 20 Gew.% Alkancarbonsäuresalze, Lösemittel, weitere anionische Tenside und 3 bis 9 Gew.% Parfümöl sowie als Transparenzkörper und Lösungsvermittler wenigstens eine Verbindung aus der Gruppe der Zucker oder Zuckerderivate und eine Verbindung aus der Gruppe der reduzierten Zucker oder deren Derivate umfasst, wobei Zuckerderivate veretherte oder veresterte Zucker oder deren glycosidische (Acetal)-Form oder substituierte Zucker oder Amino- oder und Thiozucker sind und als Alkancarbonsäuresalze Alkanmonocarbonsäuresalze mit einer Kohlenstoffkette mit zwischen 10 und 24 Kohlenstoffatomen ausgewählt werden.

Die erfindungsgemäßen Mittel sind umweltfreundlich, zeigen eine gute Reinigungsleistung, lassen sich auf einfache Weise durch Schmelzen der Komponenten und Abkühlen herstellen und weisen eine hohe Transparenz, die für ein Toilettenreinigungs- und Beduftungsmittel erforderliche Härte, ausreichende Raumbeduftung, Spülzahlen von wenigstens 150 bis 200 Spülungen/Mittel und ein ansprechendes Aussehen auf.

Im Rahmen der vorliegenden Erfindung werden unter Zucker Mono- oder Oligosaccharide, insbesondere die Polyhydroxyaldosen oder -ketosen sowie deren niedere Oligomere verstanden, wie beispielsweise die Hexosen, Pentosen oder deren Di- oder Trisaccharide. Besonders bevorzugt sind unter den Monosacchariden die Glucose, Fructose, Arabiose, Ribose, Xylose, Mannose und Galactose und unter den Disacchariden die Saccharose, Maltose, Lactose, Raffinose und Cellobiose aus natürlichen oder synthetischen Quellen, unabhängig von ihrer Fischer-Konfiguration sowie alle Epimere, die auf diesen Zuckern basieren.

Als reduzierte Formen der Zucker oder der Zuckerderivate können sämtliche unter Erhalt der C-Kette reduzierte Zucker eingesetzt werden wie die Desoxyzucker und die Zuckeralkohole.

Besonders bevorzugt werden als Zuckeralkohole Hexitole wie das Sorbitol (Glucitol), Allitol, Glucitol, Mannitol, Altritol, Iditol, Pentitole wie das Ribitol, Arabinitol und Xylitol und Tetritole wie Erythritol und Theitol, jeweils mit ihren verschiedenen Enantiomeren, eingesetzt. Auch Zuckeralkohole von Disacchariden können eingesetzt werden wie das Maltitol, Lactitol und Isomalt.

Die Kombination eines Zuckeralkohols wie insbesondere Sorbitol mit einem Mono- oder Disaccharid wie beispielsweise Saccharose ergab im Rahmen der vorliegenden Erfindung Mittel mit einer besonders hohen Transparenz.

Der Anteil an reduzierten Zuckern kann in dem Mittel bis zu 30 Gew.%, vorzugsweise 10 bis 20 Gew.%, betragen und besonders bevorzugt 15 bis 19 Gew.%.

Wird dem Mittel eine zu hohe Menge an Zucker zugesetzt, so kommt es zur Auskristallisation des Zuckers an der Oberfläche des Toilettenreinigungsmittels. Aus diesem Grund ist die Maximalmenge an Zucker in dem Mittel - je nach Art des zugesetzten Zuckers - vorzugsweise auf ca. 15 Gew.% begrenzt. Im Allgemeinen sollte der Anteil an Zucker oder Zuckerderivaten in dem Mittel bei 5 bis 15 Gew.% liegen.

Die beiden Verbindungen aus der Gruppe der Zucker, Zuckerderivate bzw. deren reduzierter Form bewirken gemeinsam die hohe Transparenz des Mittels, tragen zur Konsistenz und Härte bei, wirken als CoEmulgator und steuern zudem die Auflösegeschwindigkeit des Mittels. Der Gesamtanteil an Zucker(derivat) und reduziertem Zucker bzw. Zuckerderivaten kann bis zu 50 Gew.%, vorzugsweise bis zu 40 Gew.% und besonders bevorzugt zwischen 20 und 30 Gew.%, betragen.

Eine besonders hohe Transparenz konnte bei einem Verhältnis von Zucker zu Zuckeralkohol von etwa 1 : 1,5 bis 1 : 2,5, insbesondere etwa 1 : 2 erzielt werden.

Als Salze der Alkancarbonsäuren werden im Rahmen der vorliegenden Erfindung Alkanmonocarbonsäuresalze mit zwischen 10 und 24 Kohlenstoffatomen eingesetzt. Besonders bevorzugte Alkancarbonsäuresalze sind Salze von Laurin-, Myristin-, Palmitin-, Stearin-, Ölsäure und/oder C-Schnitte derselben aus natürlichen oder synthetischen Quellen.

Wegen der biologischen Abbaubarkeit sind insbesondere die geradzahligen, unverzweigten Alkancarbonsäuresalze mit natürlichen C-Quellen-Schnitten besonders geeignet.

Über die Auswahl der Art und Menge der Alkancarbonsäuresalze lassen sich auch der Erstarrungspunkt und die Härte einstellen, wobei mit einem höheren Anteil an Stearinsäuresalzen ein härteres, höher erstarrendes Mittel erzielt werden kann.

Das erfindungsgemäße Mittel umfasst wenigstens ein Alkancarbonsäuresalz, bevorzugt sind wenigstens zwei verschiedene und besonders bevorzugt drei verschiedene Alkancarbonsäuresalze. Eine besonders hohe Transparenz des Mittels konnte mit einem Gemisch aus Stearin-, Laurin- und Myristinsäuresalzen erzielt werden.

Die Alkancarbonsäuresalze sind vorzugsweise Alkalisalze und insbesondere Natrium- und/oder Kaliumsalze.

Im Allgemeinen werden die Alkancarbonsäuresalze aus den in der Ausgangsformulierung enthaltenen Alkancarbonsäuren und Alkalien hergestellt. Daneben ist ebenfalls möglich, der Ausgangsformulierung direkt das gewünschte Alkancarbonsäuresalz zuzusetzen.

Die nachfolgenden Konzentrationsangaben beziehen sich auf die Verwendung eines Gemisches von Alkancarbonsäuren und Alkalien in der Ausgangsformulierung.

Die Gesamtmenge an Alkancarbonsäuren in der Alkancarbonsäuren und Alkalien umfassenden Ausgangsformulierung hängt von den Anwendungseigenschaften ab und liegt in der Regel zwischen 10 Gew.% und 20 Gew.% und besonders bevorzugt zwischen 12 Gew.% und 18 Gew.%.

Soweit in der Ausgangsformulierung des Mittels mehrere verschiedene Alkancarbonsäuren enthalten sind, sollten infolge des Parfümzusatzes mehr langkettige Alkancarbonsäuren enthalten sein. Besonders bevorzugt ist, in der Ausgangsformulierung für das Mittel jeweils bis zu 8 Gew.% Myristinsäure und Laurinsäure und bis zu 20 Gew.% Stearinsäure vorzusehen, wobei die Gesamtmenge an Alkancarbonsäuren unter 20 Gew.% und bevorzugt unter 18 Gew.% liegen sollte.

Eine weitere Verbesserung der Transparenz des Mittels kann durch Zugabe von Ethercarbonsäuren oder dem entsprechenden Alkalisalz erzielt werden.

Wird das Alkancarbonsäuresalz aus Alkancarbonsäuren und Alkalien in der Ausgangsformulierung gebildet, so sollte die Ausgangsformulierung organische oder anorganische Basen wie Amine oder Alkalioxide oder Alkalihydroxide umfassen, wobei der Formulierung vorzugsweise KOH oder NaOH zugesetzt werden, die mit der/den Alkancarbonsäuren die jeweiligen Fettsäuresalze bilden. Besonders bevorzugt ist, ein Gemisch aus KOH und NaOH einzusetzen, da hierdurch sowohl eine Zunahme der Transparenz des Mittels festgestellt werden konnte, als auch der Erstarrungspunkt und die Löslichkeit des Mittels und der Fettsäuresalze beeinflusst werden kann. Im Allgemeinen sollte der Anteil an NaOH den an KOH bei weitem übersteigen.

Die Menge an Alkalien richtet sich nach der Alkancarbonsäuremenge und wird entsprechend der Säurezahl der Fettsäuren zugesetzt.

Wird zusätzlich zu den Alkancarbonsäuren noch eine weitere Säure wie beispielsweise eine Ethercarbonsäure eingesetzt, so ist es bei Erhalt der hohen Transparenz sogar ausreichend, wenn die Alkalien in geringfügigem Unterschuss in Bezug auf die Säurezahl der Alkancarbonsäuremenge zugesetzt werden.

Wie bereits erläutert kann die Ausgangformulierung anstelle von Alkancarbonsäuren und Alkalien auch bereits das gewünschte Alkancarbonsäuresalz umfassen.

Das erfindungsgemäße Mittel umfasst neben dem Alkancarbonsäuresalz weitere anionische Tenside. Zusätzlich können Tenside aus der Gruppe der nichtionischen, kationischen oder amphoteren Tenside ausgewählt werden oder Mischungen davon.

Unter den anionischen Tensiden sind insbesondere die Alkylsulfate, Alkylethersulfate, die Sulfonate wie beispielsweise die Alkylsulfonate, die Olefinsulfonate, die Alkoxyalkansulfonate, die Alkylarylsulfonate, die Sulfatester, die Alkylcarbonate, Alkylethercarboxylate, die Fettsäuretauride, die Alkylisethionate und deren Mischung bevorzugt.

Als nichtionische Tenside können beispielsweise Alkylethoxylate, ethoxylierte Alkylphenole, ethoxylierte oder propoxylierte Fettalkohole, Zuckertenside wie Alkylpolyglycoside, Polyethylenglykolether, ethoxylierte Fettsäureester, Kondensationsprodukte von Ethylenoxid mit langkettigen Aminen oder Amiden oder vergleichbaren Verbindungen, Aminoxiden, Trisiloxanalkoxylate oder deren Mischung eingesetzt werden.

Als amphotere Tenside können beispielsweise Betaine eingesetzt werden und als kationische Tenside quartäre Alkylammoniumverbindungen.

Im Falle des Einsatzes von kationischen Tensiden muss darauf geachtet werden, diese so auszuwählen, dass keine Wolkenbildung durch Bildung von unlöslichen Neutralkomplexen erfolgt.

Der Anteil an Tensiden richtet sich nach der gewünschten Reinigungs-/Schaumleistung und beträgt vorzugsweise zwischen 5 Gew.% und 25 Gew.% und insbesondere zwischen 8 Gew.% und 18 Gew.%.

Als Lösemittel können sowohl organische als auch anorganische Lösemittel eingesetzt werden. Bevorzugte Lösemittel sind Wasser und Alkohole, insbesondere mehrwertige Alkohole. Als Alkohole können beispielsweise 1,2-Propylenglykol, Dipropylenglykol, Butylenglykol, Ethylenglykol, 1,7-Heptandiol, Glycerin, Glycerinderivate, die Monoethylenglykole, die Polyethylenglykole mit einem Molekulargewicht bis zu 8000 sowie die Mono-C₁₋₄-Alkylether der vorangegangenen Verbindungen ausgewählt werden.

Der Anteil an Lösemittel sollte in dem Mittel zwischen 15 Gew.% und 40 Gew.% betragen, wobei in dem Mittel ggf. vorhandenes Parfümöl gleichzeitig auch ein Lösemittel darstellt. Vorzugsweise weist das Mittel einen relativ hohen Anteil an Alkoholen und einen relativ geringen Anteil an Wasser auf. Versuche belegen, dass durch eine Reduzierung des Wasseranteils unter Erhöhung des Zucker-/Sorbitanteils in dem Mittel eine weitere Verbesserung der Transparenz erzielt werden kann.

Vorzugsweise liegt der Wassergehalt des Mittels unter 12 Gew.%, besonders bevorzugt unter 10 Gew.%. Mittel mit solch geringen Wasseranteilen zeichnen sich durch eine hohe Transparenz bei Spülzahlen von wenigstens 200 Spülungen/Mittel aus.

Die erfindungsgemäßen Mittel zeichnen sich weiterhin auch durch eine genügende Härte bei Raumtemperatur aus. Die Mittel sind schnittfest und formstabil. Eine hinreichende Härte ist u.a. auch eine Voraussetzung dafür, dass das Mittel überhaupt die für ein Toilettenreinigungs- und Beduftungsmittel erforderlichen Spülzahlen von 150 bzw. 200 und mehr erreicht.

Unter einer hinreichenden Härte wird verstanden, dass die bei Raumtemperatur mit einem Penetrometer gemessenen Eindringtiefe nicht mehr als 3,0 mm, insbesondere nicht mehr als 2,0 mm, beträgt.

Weiterhin wurde festgestellt, dass eine besonders hohe Transparenz bei einem hohen Zucker-/Sorbitanteil und einem niederen Gehalt an Wasser und an Fettsäuren erhalten wird.

Eine Erhöhung der Transparenz des Mittels kann auch durch eine Erhöhung des Anteils an anionischen Tensiden, insbesondere der Alkylethersulfate, erzielt werden.

Das erfindungsgemäße Mittel enthält weiterhin Parfümöle in einer Menge von 3 bis 9 Gew.% und vorzugsweise von 5 bis 8 Gew.%. Hierdurch wird eine lang anhaltende kontinuierche Duftabgabe und Raumbeduftung bewirkt.

Durch die weiteren Bestandteile der erfindungsgemäßen Formulierung zeigen die Mittel trotz des hohen Parfümölgehalts eine sehr gute Transparenz, eine hinreichende Härte und Spülzahlen von 200/Mittel oder mehr.

Generell können als Parfümöle einzelne synthetische Produkte wie Ether, Ester, Aldehyde oder Ketone eingesetzt werden. Hierzu zählen beispielsweise Benzylacetat (Ester), Benzylethylether (Ether), Citral, Citronellal (Aldehyd), Citronellol oder Eugenol (Alkohole). Bevorzugt werden Mischungen eingesetzt, die ein typisches sensorisches Profil liefern. Die Parfümöle können auch aus natürlichen Quellen stammen. Die Auswahl der geeigneten Parfümöle sollte sich auch an der Polarität der eingesetzten Lösemittel orientieren.

Das erfindungsgemäße Mittel ist vorzugsweise frei von anorganischen Salzen und auch frei von Cumolsulfonat.

Zusätzlich können dem Mittel Verbindungen, die in der Lage sind, insbesondere mit Eralkaliionen Komplexe zu bilden, zugesetzt werden. Zu diesen Verbindungen zählen Polycarbonsäuren wie Adipinsäure, Nitrilotriessigsäure, Weinsäure sowie die Stoffklasse der Aminoalkanphosphonate und deren Alkalimetallsalze sowie polymere Polycarboxylate.

Wahlweise kann das Mittel auch noch Bleichmittel, Oxidationsmittel, Korrosionsschutzmittel, Nano-Partikel, keimtötende Mittel, dekorative Farbgranulate und/oder Polymere wie PVA, Cellulose oder Arylamide umfassen. Auch der Zusatz von Farbstoffen ist möglich, wobei bei der Zugabe von Farbstoffen allerdings bedacht werden muss, dass der Transparenzeindruck infolge von zunehmender Lichtabsorption durch die Farbstoffe abnimmt.

Die Komponenten des erfindungsgemäßen Mittels werden vorzugsweise zusammen geschmolzen und dann in eine beliebige, geschlossene Form mit wenigstens einer Öffnung gegossen, um dort zu erstarren. Die Befüllung kann mit den bekannten diskontinuierlich und kontinuierlich arbeitenden Verfahren erfolgen. Im Vergleich zu den bekannten extrudierten Toilettenreinigungsmitteln, die aufwändige Mischer und Extruder erforderlich machen, kann das erfindungsgemäße optisch ansprechende Mittel somit in einem weitaus einfacheren Verfahren hergestellt werden.

Der Erstarrungspunkt des erfindungsgemäßen Mittels liegt in Abhängigkeit vom Wasser- und Fettsäureanteil zwischen 45 °C bis 70 °C und kann damit für die unterschiedlichsten Anwendungs- und Temperaturbereiche eingestellt werden.

Die Transparenz der erfindungsgemäßen Mittel ist dauerhaft und es kommt nicht zu der aus dem Stand der Technik bekannten Eintrübung infolge der Kristallisation oder Flockenbildung der Fettsäuresalze oder der Zucker(verbindungen) während des Lagerns. Auch zeigen die erfindungsgemäßen Mittel bereits nach dem Erstarren die gewünschte Transparenz, so dass das aus dem Stand der Technik bekannte "Reifen" nicht erforderlich ist.

Eine Variante der vorliegenden Erfindung sieht vor, in das hochtransparente Mittel Gegenstände wie beispielsweise Tierfiguren oder andere ansprechende Figuren oder eine Marke einzubetten, um die Optik des Mittels weiter zu verbessern und den Wiedererkennungswert zu erhöhen.

Die Behältnisse zur Aufnahme des Mittels, beispielsweise für den WC-Rand, sind dann so umzugestalten, dass der Verbraucher das optisch ansprechende Mittel auch wahrnehmen kann.

Ebenfalls ist daran gedacht, die hochtransparente Phase des vorliegenden Mittels mit anderen duftenden oder reinigenden Phasen zu kombinieren, beispielsweise die hochtransparente reinigende Phase des vorliegenden Mittels mit einer gelförmige Duftstoffe enthaltenden Phase zu einem insgesamt transparenten gut reinigenden und stark und langanhaltend duftenden Mittel zu kombinieren. Falls gewünscht kann hierbei die Gelphase eingefärbt werden oder gar ein Mittel mit einer transparenten Seifenphase und zwei verschiedenartig eingefärbten Gelphasen bereitgestellt werden.

Eine solche Gelphase kann beispielsweise, wie in der EP 1 418 225 oder der DE 197 10 635 A1 beschrieben, hergestellt werden.

Die Erfindung wird nachfolgend anhand von Ausführungsbeispielen und Vergleichsversuchen näher beschrieben.

In Tabelle 1 ist die erfindungsgemäße Rezeptur 18 verschiedenen Vergleichsversuchen gegenübergestellt.

Die Herstellung der Mittel erfolgte derart, dass die einzelnen Komponenten zusammen geschmolzen wurden und dann in eine Form gegossen wurden, wo sie erstarrten. Nach Abkühlung auf Raumtemperatur sind die erhaltenen Mittel fest.

Die in den Rezepturen in Tabelle 1 eingesetzten Rohstoffe sind in der nachfolgenden Tabelle 2 aufgeführt.

Die erfindungsgemäße Rezeptur 18, die 5% Gew.% Parfumöl umfasst, zeichnet sich über die gesamte Lebensdauer durch die Freisetzung von Duftstoffen aus.

In Tabelle 1 sind zudem die Schaumwerte, Oberflächenspannung, das Abspülverhalten, der Dufteindruck und die Transparenz der einzelnen Rezepturen aufgelistet, die - wie nachfolgend beschrieben - bestimmt wurden.

In Tabelle 3 sind die Härten einzelner Proben aufgelistet.

### 1. Schaumwerte:

### 1a) Herstellung der Stammlösung:

In einen 1 I Meßkolben werden 1,00 g Produkt eingewogen, mit demineralisiertem Wasser aufgelöst. Dazu werden 20 ml Calciumchlorid-Lösung gegeben, auf 20°C temperiert und mit demineralisiertem Wasser aufgefüllt.

Zur Herstellung der Calciumchlorid-Lösung werden 16,7 g Calciumchlorid Dihydrat p.a. in 21 demineralisiertem Wasser gelöst.

Die so hergestellte Stammlösung entspricht einer Wasserhärte von 6,4 °dH.

### 1b) Schaumzahl:

Man überführt 100 ml der auf 20 °C temperierten Stammlösung in einen 250 ml Mischzylinder und verschließt diese mit einem PTFE-Stopfen. Dann wird der Zylinder zwanzigmal hin und her gewendet (20 mal auf den Kopf gestellt). Nach jeweils: 30 sec/5 min/30 min wird das erzeugte Schaumvolumen (ml) abgelesen und notiert.

### 2. Bestimmung der Oberflächenspannung:

### Gerät: Krüss BP 2 Blasendruckmeßgerät

### Prüfung Blindwert:

Mit dem Ufaryl DL 90 C wird eine 0,1%ige -Lösung hergestellt, bei 20°C dreimal eine Meßkurve mit dem vorher ermittelten Kapillardurchmesser aufgenommen und der Mittelwert gebildet. Die Abweichung vom Mittelwert darf maximal 1 mN/m sein.

Die Erstprüfung wird als Urwert genommen, alle Folgeprüfungen werden mit dem Urwert verglichen (50/100/500 ms). Bei Abweichung größer als ±2 mN/m ist die Kapillare durch eine neue auszutauschen.

### Messung:

Von einer 0,1%-igen Lösung (20 °C) wird die Oberflächenspannung in Abhängigkeit vom Alter der Oberfläche ermittelt. Betrachtet wird dabei die Oberflächenspannung nach 100, 500, 1000 ms.

### 3. Bestimmung des Abspülverhalten:

### Spülvolumen pro Spülung: 8 I

### Spülrhythmus: 19 Spülungen am Tag nach einem definierten Schema.

Spülwassertemperatur: Die Spülwassertemperatur beträgt während der kurzen Spülfrequenzen (z.B. zwischen 3. und 5. Spülung) 13° - 14° C, während der langen Ruheperioden (z.B. zwischen 19. und 1. Spülung) 15° - 16°C. Die Temperatur des Spülwassers wird an jedem Werktag überprüft und notiert.

### 4. Bestimmung des Dufteindruckes:

Tägliche olfaktorische Bestimmung des Geruchs durch Anriechen der Probe in der Testtoilette.

### 5. Bestimmung der Transparenz:

Als Maß für die Transparenz der Mittel wurde überprüft, inwieweit noch Schriften mit abnehmender Größe durch verschiedene Schichtdicken des Mittels lesbar sind.

Zur Feststellung der Transparenz des Mittels wurden die Ansätze in Aluminiumformen (Innenmaß: LxBxH = 5cm x 2cm x ca. 8 cm) gegossen, über Nacht abgekühlt und am nächsten Tag aus der Form gepresst.

Beurteilt wurde die Lesbarkeit einer Schrift von 3 bzw. 5 Pixeln Höhe, die auf einem HP Laserjet 2100 erstellt wurde. Die höchste Transparenz konnte mit der nicht erfindungsgemäßen parfümfreien Rezeptur 24 erzielt werden, die sich zudem auch noch durch relativ wenige Einzelbestandteile auszeichnet.

### 6. Bestimmung der Härte:

Die Härtebestimmung erfolgte mit einer Nadelpenetrationsmessung mit dem Messgerät PNR 10 der Fa. Petrotest (vormals Sommer und Runge KG, Berlin). Die Messtemperatur betrug 19,5 °C, die Prüflast (Standard Prüfkegel inklusive Fallstange) wog 150 g.

Die Nadelspitze wurde bis kurz vor die Oberfläche des Prüflings gefahren und dann über die Geräteautomatik ausgelöst. Abgelesen werden die Eindringtiefen in mm.

**Tabelle 1:**

| | Rezepturen | | | | | | | Vergleich |
|---|---|---|---|---|---|---|---|---|
| | 2/2a | 2b | 11 | 17 | 18 | 19 | 24 | US 2001/0 044394 |
| | | | | | | | | |
| 1,2 Propylenglycol | 21,5 | 21,5 | 21,5 | 15 | 16,5 | 21,5 | 16 | 20 |
| Stearinsäure | 9,2 | 9,2 | 9,2 | 13 | 9,2 | 16 | 9,2 | 13 |
| Laurinsäure | 5 | 5 | 5 | 0 | 5 | 0 | 5 | 0 |
| Myristensäure | 1,8 | 1,8 | 1,8 | 6 | 1,8 | 0 | 1,8 | 6 |
| 1-Hydroxy-1,1-diphosphonsäure | 0,5 | 0,1 | 0 | 0 | 0 | 0 | 0 | 0 |
| Wasser | 9 | 9 | 9 | 15,8 | 9 | 9 | 9 | 15,8 |
| NaOH | 2,5 | 2,5 | 2,5 | 3 | 2,5 | 2,5 | 2,5 | 3 |
| Ethylendiamin-tetraessigsäuredinatriumsalz; flüssig | 0,06 | 0,06 | 0 | 0 | 0 | 0 | 0 | 0 |
| Glycerin 86,5 % | 9 | 9 | 9 | 13 | 9 | 9 | 9 | 13 |
| C12-C14 Alky lethersulfat 1,3 EO | 10 | 10 | 10 | 12,25 | 10 | 10 | 0 | 12,25 |
| C12-C14 Alky lethersulfat 2,5 EO | 0 | 0 | 0 | 0 | 0 | 0 | 15,5 | 0 |
| Laurysulfat (94%) | 3,5 | 3,5 | 3,5 | 0 | 3,5 | 3,5 | 3,5 | 0 |
| Sorbit, kristallin | 17,8 | 17,8 | 17,8 | 10,5 | 17,8 | 17,8 | 17,8 | 10,5 |
| NaCl | 1 | 1 | 0 | 0 | 0 | 0 | 0 | 0 |
| hydriertes Rizi nusöl | 0,1 | 0,1 | 0 | 0 | 0 | 0 | 0 | 0 |
| Saccharose | 9 | 9 | 9 | 0 | 9 | 9 | 9 | 0 |
| Kalilauge, 50% | 0,66 | 0,66 | 0,66 | 0 | 0,66 | 0,66 | 0,66 | 0 |
| Laurylethercar bonsäure | 0 | 1 | 0 | 0 | 0 | 0 | 0 | 0 |
| Cocoylisethionat | 0 | 0 | 0 | 5 | 0 | 0 | 0 | 5 |
| TEA | 0 | 0 | 0 | 1,5 | 0 | 0 | 0 | 1,5 |
| Parfüm | 0 | 0 | 0 | 5 | 5 | 0 | 0 | 0 |
| | | | | | | | | |
| Summe | 100,62 | 101,22 | 98,96 | 100,1 | 98,96 | 98,96 | 98,96 | 100,05 |
| | | | | | | | | |
| Zucker/Sorbit | 5,06 | 5,06 | 5,06 | 0,00 | 5,06 | 5,06 | 5,06 | 0,00 |
| Zucker + Sorbit | 26,800 | 26,800 | 26,800 | 10,500 | 26,800 | 26,800 | 26,800 | 10,500 |
| Fettsäuren | 16,000 | 16,000 | 16,000 | 19,000 | 16,000 | 16,000 | 16,000 | 19,000 |
| Wasser | 9,000 | 9,000 | 9,000 | 15,800 | 9,000 | 9,000 | 9,000 | 15,800 |
| Propylenglycol | 21,500 | 21,500 | 21,500 | 15,000 | 16,500 | 21,500 | 16,000 | 20,000 |
| Gesamtlauge (auf NaOH) | 2,736 | 2,736 | 2,736 | 3,402 | 2,736 | 2,736 | 2,736 | 3,402 |
| FS/Lauge | 5,849 | 5,849 | 5,849 | 5,585 | 5,849 | 5,849 | 5,849 | 5,585 |
| FS/Wasser | 1,778 | 1,778 | 1,778 | 1,203 | 1,778 | 1,778 | 1,778 | 1,203 |
| Gesamttensid | 10,500 | 10,500 | 10,500 | 13,575 | 10,500 | 10,500 | 14,350 | 13,575 |
| Erstarrungspunkt | n.b. | n.b. | 51-52 | n.b. | 46-47 °C | 69-70 | 54-55°C | 62-63 |
| Schaum [ml]; | | | | | | | | |
| (0,1%); | | | | | | | | |
| 30sec/5min/30 min | n.b. | n.b. | 2/2/2 | n.b. | 5/5/1 | n.b. | 70,2/6 5/61 | 55/50/ 35 |
| Oberflächenspannung [mN/m]; | | | | | | | | |
| (0,1%); | | | 69,9/ | | 69,4/ | | | 70,2/ |
| 100ms/500ms/10 | | | 68,2/ | | 64 | | 70,2/6 | 67/ |
| 00ms | n.b. | n.b. | 67,3 | n.b. | 61 | n.b. | 5/61 | 64 |
| Abspülversuche [Anzahl Spülungen mit 8 Liter Wasser) | n.b. | n.b. | > 250 | > 250 | 267 | n.b. | n.b. | n.b. |
| Duft [Anzahl] an eindeutig guter Geruchswahrnehmung | n.b. | n.b. | n.b. | n.b. | 267 | n.b. | n.b. | n.b. |
| Lesbarkeit durch eine 5 er Schrift/Schichtdick e 5 cm | gerade noch | gut | gut | nicht mehr | Gut | gerade noch | sehr gut | nicht mehr |
| Lesbarkeit durch eine 3 er Schrift / Schichtdicke 5 cm | nicht mehr | nicht mehr | gerade noch | nicht mehr | Gut | nicht mehr | sehr gut | nicht mehr |

| | | | | | | | | |
|---|---|---|---|---|---|---|---|---|
| FS: = Fettsäure n.b.: = nicht bestimmt Einwaagen in Gramm | | | | | | | | |

**Tabelle 2:**

| Rohstoff | Handelsbezeichnung | Lieferant | Bemerkungen |
|---|---|---|---|
| 1,2 Propylenglycol | 1,2 Propylenglycol | Biesterfeld | |
| Stearinsäure | Prifrac 2981 | Uniquema | |
| Laurinsäure | Prifrac 2922 | Uniquema | |
| Myristensäure | Prifrac 2942 | Uniquema | |
| 1-Hydroxy-1,1-diphosphonsäure | Sequion 10 H 60 | Polygon | |
| Wasser | ./. | | enthärtet, kalt |
| NaOH | Natriumhydroxid | Häffner | Microprills |
| Ethylendiamintetraessigsäuredinatriumsalz; fest | Trilon BD | BASF | |
| Glycerin 86,5 % C12-C14 Alkylethersulfat 1,3 | Glycerin 86,5 % | Biesterfeld | |
| EO | Texapon SP N 70 | Cognis | |
| C12-C14 Alkylethersulfat 2,5 EO | Texapon N 70 | Cognis | |
| Laurysulfat (94%) | Tensopol USP 94 | Manro | |
| Sorbit, kristallin | | Suga | |
| NaCl | ./. | | handelsübliches Kochsalz |
| Hydriertes Rizinusöl | Cremophor RLM 410 | BASF | |
| Saccharose | Kristallzucker | Südzucker | handelüblicher Kristallzucker, raffiniert |
| Kalilauge, 50% | | Brenntag | |
| Laurylethercarbonsäure | Akypo RLM 100 | Kao Chemicals | |
| Cocoylisethionat | Hostapon KA | Clariant | |
| TEA | Triethaniolamin, rein | BASF | |
| Parfüm | Orange Fun; # 561415 | Quest | |
| | | | |
| | | | |
| | | | |

**Tabelle 3:**

| Versuche | Us 2001/ 0044394 | V2 | 2b | 11 | 24 | 17 | 18 | 19 |
|---|---|---|---|---|---|---|---|---|
| Messwerte [mm] | 1,33 | 1,48 | 1,71 | 1,58 | 1,8 | 1,45 | 2,14 | 1,84 |
| | 1,44 | 1,51 | 1,62 | 1,62 | 1,87 | 1,58 | 2,64 | 1,55 |
| | 1,62 | 1,54 | 1,59 | 1,81 | 1,91 | 1,73 | 2,16 | 1,62 |
| gemittelt | 1,46 | 1,51 | 1,64 | 1,67 | 1,86 | 1,59 | 2,31 | 1,67 |

## Patentansprüche

1. Transparentes stückförmiges Toilettenreinigungs- und Beduftungsmittel, welches Mittel umfasst:
wenigstens ein Alkancarbonsäuresalz, Lösemittel, weitere anionische Tenside, Parfümöl und wenigstens eine Verbindung aus der Gruppe der Zucker und Zuckerderivate und wenigstens eine Verbindung aus der Gruppe der reduzierten Zucker und Zuckerderivate, wobei der Anteil an Alkancarbonsäuresalzen 10,0 bis 22,0 Gew.% und der Anteil an Parfumöl 3,0 bis 9,0 Gew.% beträgt und Zucker Mono- oder Oligosaccharide und Zuckerderivate veretherte oder veresterte Zucker oder deren glycosidische (Acetal)-Form oder substituierte Zucker oder Aminozucker oder Thiozucker sind und als Alkancarbonsäuresalze Alkanmonocarbonsäuresalze mit einer Kohlenstoffkette mit zwischen 10 und 24 Kohlenstoffatomen ausgewählt werden.

2. Mittel nach Anspruch 1, **dadurch gekennzeichnet, dass** die Zucker aus der Gruppe der Hexosen, Pentosen oder deren Di- oder Trisaccharide ausgewählt werden.

3. Mittel nach Anspruch 2, **dadurch gekennzeichnet, dass** die Zucker aus der Gruppe Glucose, Fructose, Arabiose, Ribose, Xylose, Mannose, Galactose, Saccharose, Maltose, Lactose, Raffinose und Cellobiose aus natürlichen oder synthetischen Quellen, unabhängig von ihrer Fischer-Konfiguration sowie alle Epimeren, die auf diesen Zuckern basieren, ausgewählt werden.

4. Mittel nach Anspruch 1, **dadurch gekennzeichnet, dass** die reduzierten Zucker oder Zuckerderivate unter Erhalt der C-Kette reduzierte Zucker, insbesondere Desoxyzucker oder Zuckeralkohole, sind.

5. Mittel nach Anspruch 4, **dadurch gekennzeichnet, dass** die Zuckeralkohole aus der aus Sorbitol (Glucitol), Allitol, Glucitol, Mannitol, Altritol, Iditol, den Pentitolen wie Ribitol, Arabinitol und Xylitol und den Tetritolen wie Erythritol und Theitol, jeweils mit ihren verschiedenen Enantiomeren, und den Disacchariden Maltitol, Lacitol und Isomalt bestehenden Gruppe ausgewählt werden.

6. Mittel nach einem der vorangegangenen Ansprüche, **dadurch gekennzeichnet, dass** der Anteil an reduziertem Zuckern in dem Mittel bis zu 30 Gew.%, vorzugsweise 10 bis 20 Gew.%, und besonders bevorzugt 15 bis 19 Gew.%, beträgt.

7. Mittel nach einem der vorangegangenen Ansprüche, **dadurch gekennzeichnet, dass** der Gesamtanteil an Zucker, reduziertem Zucker bzw. deren Derivaten bis zu 50 Gew.%, vorzugsweise bis zu 40 Gew.% und besonders bevorzugt zwischen 20 und 30 Gew.%, beträgt.

8. Mittel nach einem der vorangegangenen Ansprüche, **dadurch gekennzeichnet, dass** das Verhältnis von Zucker zu Zuckeralkohol 1 : 1,5 bis 1 : 2,5 und vorzugsweise etwa 1 : 2, beträgt.

9. Mittel nach einem der vorangegangenen Ansprüche, **dadurch gekennzeichnet, dass** die Alkancarbonsäuresalze aus der Gruppe der Salze der Laurin-, Myristin-, Palmitin-, Stearin-, Ölsäure und/oder den C-Schnitten derselben aus natürlichen oder synthetischen Quellen ausgewählt werden.

10. Mittel nach einem der vorangegangenen Ansprüche, **dadurch gekennzeichnet, dass** die Gesamtmenge an Alkancarbonsäuren in der Alkancarbonsäuren und Alkalien zur Bildung von Alkancarbonsäuresalzen umfassenden Ausgangsformulierung zwischen 10 Gew.% und 20 Gew.% und besonders bevorzugt zwischen 12 Gew.% und 18 Gew.%, beträgt.

11. Mittel nach Anspruch 10, **dadurch gekennzeichnet, dass** die Ausgangsformulierung als Alkalien anorganische oder organische Basen, insbesondere aus der Gruppe der Alkalihydroxide oder -oxide, umfasst.

12. Mittel nach einem der vorangegangenen Ansprüche, **dadurch gekennzeichnet, dass** das Mittel weitere Tenside aus der Gruppe der nichtionischen, kationischen oder amphoteren Tenside umfasst.

13. Mittel nach einem der vorangegangenen Ansprüche, **dadurch gekennzeichnet, dass** die Lösemittel Wasser und/oder Alkohole, insbesondere mehrwertige Alkohole aus der Gruppe 1,2-Propylenglykol, Dipropylenglykol, Butylenglykol, Ethylenglykol, 1,7-Heptandiol, Glycerin, Glycerinderivate, der Monoethylenglykole, der Polyethylenglykole und/oder die Mono-C₁₋₄-Alkylether der vorangegangenen Verbindungen sind.

14. Mittel nach einem der vorangegangenen Ansprüche, **dadurch gekennzeichnet, dass** die Spülzahl des Mittels wenigstens 150 und vorzugsweise wenigstens 200 beträgt.

15. Verfahren zur Herstellung eines Mittels nach einem der Ansprüche 1 bis 14, **dadurch gekennzeichnet, dass** die einzelnen Komponenten zusammen geschmolzen und dann in eine entsprechende Form gegossen werden, um dort zu erstarren.

16. Verfahren nach Anspruch 15, **dadurch gekennzeichnet, dass** es ein kontinuierlich oder ein diskontinuierlich arbeitendes Verfahren ist.

## Claims

1. Transparent bar-shaped toilet cleaning and deodorization composition, which composition comprises:
at least one alkanecarboxylic acid salt, solvent(s), further anionic surfactants, perfume oil and at least one compound from the group of sugars and sugar derivatives and at least one compound from the group of reduced sugars and sugar derivatives, where the fraction of alkanecarboxylic acid salts is 10.0 to 22.0% by weight and the fraction of perfume oil is 3.0 to 9.0% by weight and sugars are mono- or oligosaccharides and sugar derivatives are etherified or esterified sugars or their glycosidic (acetal) form or substituted sugars or amino sugars or thio sugars, and the alkanecarboxylic acid salts selected are alkanemonocarboxylic acid salts with a carbon chain having between 10 and 24 carbon atoms.

2. Composition according to Claim 1, **characterized in that** the sugars are selected from the group of hexoses, pentoses or their di- or trisaccharides.

3. Composition according to Claim 2, **characterized in that** the sugars are selected from the group consisting of glucose, fructose, arabiose, ribose, xylose, mannose, galactose, sucrose, maltose, lactose, raffinose and cellobiose from natural or synthetic sources, independently of their Fischer configuration, and also all epimers which are based on these sugars.

4. Composition according to Claim 1, **characterized in that** the reduced sugars or sugar derivatives are sugars which are reduced by maintaining the carbon chain, in particular deoxy sugars or sugar alcohols.

5. Composition according to Claim 4, **characterized in that** the sugar alcohols are selected from the group consisting of sorbitol (glucitol), allitol, glucitol, mannitol, altritol, iditol, the pentitols such as ribitol, arabinitol and xylitol and the tetritols such as erythritol and theitol, in each case with their various enantiomers, and the disaccharides maltitol, lacitol and isomalt.

6. Composition according to one of the preceding claims, **characterized in that** the fraction of reduced sugar in the composition is up to 30% by weight, preferably 10 to 20% by weight, and particularly preferably 15 to 19% by weight.

7. Composition according to one of the preceding claims, **characterized in that** the total fraction of sugar, reduced sugar or derivatives thereof is up to 50% by weight, preferably up to 40% by weight and particularly preferably between 20 and 30% by weight.

8. Composition according to one of the preceding claims, **characterized in that** the ratio of sugar to sugar alcohol is 1:1.5 to 1:2.5 and preferably about 1:2.

9. Composition according to one of the preceding claims, **characterized in that** the alkanecarboxylic acid salts are selected from the group of the salts of lauric acid, myristic acid, palmitic acid, stearic acid, oleic acid and/or the carbon cuts of the same from natural or synthetic sources.

10. Composition according to one of the preceding claims, **characterized in that** the total amount of alkanecarboxylic acids in the alkanecarboxylic acids and alkalis for the formation of starting formulation comprising alkanecarboxylic acid salts is between 10% by weight and 20% by weight and particularly preferably between 12% by weight and 18% by weight.

11. Composition according to Claim 10, **characterized in that** the starting formulation comprises, as alkalis, inorganic or organic bases, in particular from the group of alkali metal hydroxides or oxides.

12. Composition according to one of the preceding claims, **characterized in that** the composition comprises further surfactants from the group of nonionic, cationic or amphoteric surfactants.

13. Composition according to one of the preceding claims, **characterized in that** the solvents are water and/or alcohols, in particular polyhydric alcohols from the group consisting of 1,2-propylene glycol, dipropylene glycol, butylene glycol, ethylene glycol, 1,7-heptanediol, glyceryl, glyceryl derivatives, of monoethylene glycols, of polyethylene glycols and/or the mono-C₁₋₄-alkyl ethers of the preceding compounds.

14. Composition according to one of the preceding claims, **characterized in that** the flush number of the composition is at least 150 and preferably at least 200.

15. Process for the preparation of a composition according to one of Claims 1 to 14, **characterized in that** the individual components are melted together and then poured into an appropriate mould in order to solidify there.

16. Process according to Claim 15, **characterized in that** it is a continuous or discontinuous process.

## Revendications

1. Agent de nettoyage et de parfum de toilettes en morceau, transparent, l'agent comprenant :
au moins un sel d'acide alcanecarboxylique, du (des) solvant(s), d'autres agents tensioactifs anioniques, de l'huile parfumée et au moins un composé du groupe formé par les sucres et les dérivés du sucre et au moins un composé du groupe des sucres réduits et des dérivés des sucres réduits, la proportion de sels d'acide alcanecarboxylique étant de 10,0 à 22,0% en poids et la proportion d'huile parfumée étant de 3,0 à 9,0% en poids et les sucres étant des monosaccharides ou des oligosaccharides et des dérivés de sucres éthérifiés ou estérifiés ou leur forme (acétal) glycosidique ou des sucres substitués ou des aminosucres ou des thiosucres et des sels d'acides alcanemonocarboxyliques présentant une chaîne carbonée comprenant entre 10 et 24 atomes de carbone étant choisis comme sels d'acide alcanecarboxylique.

2. Agent selon la revendication 1, **caractérisé en ce que** les sucres sont choisis dans le groupe des hexoses, des pentoses ou de leurs disaccharides ou trisaccharides.

3. Agent selon la revendication 2, **caractérisé en ce que** les sucres sont choisis dans le groupe formé par le glucose, le fructose, l'arabiose, le ribose, le xylose, le mannose, le galactose, le saccharose, le maltose, le lactose, le raffinose et le cellobiose provenant de sources naturelles ou synthétiques, indépendamment de leur configuration de Fisher, ainsi que tous les épimères, qui sont à base de ces sucres.

4. Agent selon la revendication 1, **caractérisé en ce que** les sucres réduits ou dérivés des sucres réduits sont des sucres réduits avec conservation de la chaîne carbonée, en particulier les sucres désoxy ou les alcools de sucre.

5. Agent selon la revendication 4, **caractérisé en ce que** les alcools de sucre sont choisis dans le groupe constitué par le sorbitol (glucitol), l'allitol, le glucitol, le mannitol, l'altritol, l'iditol, les pentitols tels que le ribitol, l'arabinitol et le xylitol et les tétritols tels que l'érythritol et le théitol, à chaque fois avec leurs différents énantiomères, et les disaccharides maltitol, lacitol et isomalte.

6. Agent selon l'une quelconque des revendications précédentes, **caractérisé en ce que** la proportion de sucres réduits dans l'agent est de jusqu'à 30% en poids, de préférence de 10 à 20% en poids, et de manière particulièrement préférée de 15 à 19% en poids.

7. Agent selon l'une quelconque des revendications précédentes, **caractérisé en ce que** la proportion totale de sucres, de sucres réduits ou de leurs dérivés est de jusqu'à 50% en poids, de préférence de jusqu'à 40% en poids, et de manière particulièrement préférée entre 20 et 30% en poids.

8. Agent selon l'une quelconque des revendications précédentes, **caractérisé en ce que** le rapport de sucre à alcool de sucre est de 1:1,5 à 1:2,5 et de préférence d'environ 1:2.

9. Agent selon l'une quelconque des revendications précédentes, **caractérisé en ce que** les sels d'acide alcanecarboxylique sont choisis dans le groupe des sels de l'acide laurique, myristique, palmitique, stéarique, oléique et/ou des fractions carbonées de ceux-ci, provenant de sources naturelles ou synthétiques.

10. Agent selon l'une quelconque des revendications précédentes, **caractérisé en ce que** la quantité totale d'acides alcanecarboxyliques dans la formulation de départ comprenant les acides alcanecarboxyliques et les alcalis pour la formation des sels d'acide alcanecarboxyliques est entre 10% en poids et 20% en poids et de manière particulièrement préférée entre 12% en poids et 18% en poids.

11. Agent selon la revendication 10, **caractérisé en ce que** la formulation de départ comprend, comme alcalis, des bases inorganiques ou organiques, en particulier du groupe des hydroxydes ou oxydes de métal alcalin.

12. Agent selon l'une quelconque des revendications précédentes, **caractérisé en ce que** l'agent contient d'autres agents tensioactifs du groupe des agents tensioactifs non ioniques, cationiques ou amphotères.

13. Agent selon l'une quelconque des revendications précédentes, **caractérisé en ce que** les solvants sont l'eau et/ou des alcools, en particulier des alcools polyvalents du groupe formé par le 1,2-propylèneglycol, le dipropylèneglycol, le butylèneglycol, l'éthylèneglycol, le 1,7-heptanediol, le glycérol, les dérivés du glycérol, les monoéthylèneglycols, les polyéthylèneglycols et/ou les mono-C₁₋₄-alkyléthers des composés susmentionnés.

14. Agent selon l'une quelconque des revendications précédentes, **caractérisé en ce que** le nombre de rinçages de l'agent est d'au moins 150 et de préférence d'au moins 200.

15. Procédé pour la préparation d'un agent selon l'une quelconque des revendications 1 à 14, **caractérisé en ce que** les différents composants sont fondus ensemble, puis versés dans un moule correspondant, pour y durcir.

16. Procédé selon la revendication 15, **caractérisé en ce qu'**il s'agit d'un procédé réalisé de manière continue ou discontinue.
